# FASCICULE DE BREVET EUROPEEN

(11) **EP 0 627 403 B1**
(45) Date de publication et mention de la délivrance du brevet: **18.12.1996**
(21) Numéro de dépôt: 94401041.2
(22) Date de dépôt: 10.05.1994
(51) Int. Cl.: C07C 59/52, C07C 51/377

(54) **Procédé de préparation d'acides hydroxyphénylacétiques**
Verfahren zur Herstellung von Hydroxyphenylessigsäuren
Method for the production of hydroxyphenylacetic acids

(30) Priorité: 28.05.1993 FR 9306433
(43) Date de publication de la demande: 07.12.1994
(73) Titulaire: SOCIETE FRANCAISE HOECHST Société anonyme dite:, F-92800 Puteaux (FR)
(72) Inventeur: Vallejos, Jean-Claude, F-75018 Paris (FR); Christidis, Yani, F-75019 Paris (FR)
(74) Mandataire: Rinuy, Santarelli

(56) Documents cités:
- EP-A- 0 526 672
- EP-A- 0 554 636
- 'Houben-Weyl: Methoden der Organischen Chemie, 4ième édition, tome IV/1d, partie II' , GEORG THIEME VERLAG, STUTTGART, DE * page 561 *

## Description

La présente demande concerne un procédé de préparation d'acides hydroxyphénylacétiques.

Les acides o-, m- et p-hydroxyphénylacétiques, substitués ou non sur le noyau aromatique par un ou plusieurs substituants choisis parmi les halogènes ou les radicaux alcoyles ou alcoxyles sont des matières premières couramment utilisées en synthèse organique pour accéder à des produits présentant d'intéressantes propriétés physiologiques.

On connaît de très nombreux procédés pour obtenir ces acides hydroxyphénylacétiques. Parmi ceux-là, signalons l'hydrogènolyse soit par voie catalytique, soit par voie chimique, des acides hydroxymandéliques correspondants qui peuvent être issus de l'hydrolyse de l'hydroxymandélonitrile correspondant, ou pour certains d'entre eux, de la condensation de l'acide glyoxylique avec le phénol correspondant (cf Beil., 10, 410, 10, I, 199, 10-III, 1471 et 1474, 10-V, 1518, EP-A-536 960, FR-A-2 440 350, 2 427 322, 2 495 137, 2 638 740). Les procédés connus d'hydrogénolyse des acides hydroxymandéliques en acides hydroxyphénylacétiques correspondants présentent toutefois au niveau industriel de sérieux inconvénients en raison notamment du prix élevé de certains agents réactifs utilisés tels que l'acide iodhydrique, l'iode, le phosphore rouge, l'acide phosphoreux ou les sels d'étain ou de chrome (FR-A-2 426 669, 2 445 311, 2 588 869, GB-A-2 078 718, US-A-5 145 994, EP-A-028 375, 032 374, 526 672 et 224 401, JP-A-50/092 344, 58-052 242, Beil. 10, 187, 189, 190, 10 ,I, 81, 82, 10, II, 112, 10, III, 422, 428, 430, 10, IV, 536, 541, 543).

Afin d'obvier à ces inconvénients, la demanderesse a découvert avec étonnement un nouveau procédé de préparation des acides hydroxyphénylacétiques.

La présente invention a pour objet un procédé de préparation d'un acide hydroxyphénylacétique de formule (I) 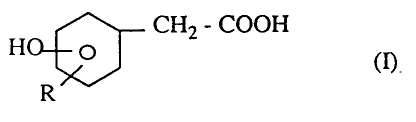
dans laquelle R représente un atome d'hydrogène, d'halogène ou un groupe alcoyle ou alcoxyle.

Le terme alcoyle peut désigner, par exemple, un radical en C₁-C₅, tel un radical méthyle, éthyle, n-propyle, isopropyle, n-butyle, isobutyle, sec-butyle, tertiobutyle, pentyle.

le terme alcoxyle peut désigner, par exemple, un radical en C₁-C₅, tel un radical méthoxyle, éthoxyle, propoxyle, isopropoxyle, butoxyle, isobutoxyle, sec-butoxyle, tertiobutoxyle, pentyloxyle.

Le terme halogène désigne le chlore ou le brome.

Selon l'invention, le procédé de préparation des acides hydroxyphénylacétiques de formule (I) et de leurs sels est caractérisé par le fait que l'on fait réagir en milieu aqueux, à une température supérieure ou égale à 50°C, l'acide hydroxymandélique correspondant, libre ou salifié de formule (II) 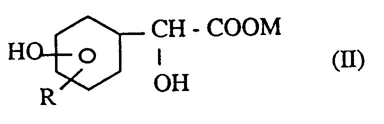
dans laquelle R a la signification donnée précédemment et M représente un atome d'hydrogène, de sodium, de potassium ou un groupement ammonium, avec de l'acide formique en présence de quantités catalytiques d'un dérivé oxygéné du soufre présentant un degré d'oxydation de 2 à 4, pour obtenir l'acide hydroxyphénylacétique de formule (I) que l'on isole ou, si désiré, on salifie selon les méthodes usuelles.

Parmi les dérivés oxygénés du soufre présentant un degré d'oxydation de 2 à 4, on peut citer l'hydrogènosulfite de sodium ou de potassium, le sulfite de sodium ou de potassium, le disulfite de sodium ou de potassium, le thiosulfate de sodium ou de potassium, le dithionite de sodium ou de potassium, le formaldéhyde sulfoxylate de sodium, le dioxyde de soufre.

La demande de brevet EP-A-554 636, publiée le 11.08.93, décrit, inter alia, un procédé de préparation de l'acide p-hydroxyphénylacétique par réaction, dans un solvant agent, de l'acide formique sur l'acide p-hydroxy-mandélique, en présence d'un système catalytique constitué par du palladium dépore sur du charbon et des ions Cl⁻.

Dans des conditions préférentielles de mise en oeuvre de l'invention, le procédé ci-dessus décrit est réalisé de la manière suivante :
- à une température comprise entre 75 et 150°C,
- à une pression de 2 à 10 bars,
- en présence de quantités catalytiques de disulfite de sodium.

Dans des conditions encore plus préférentielles, le procédé ci-dessus décrit est mis en oeuvre de la manière suivante :
- à une température comprise entre 100 et 130°C et
- à une pression de 4 à 5 bars.

Dans d'autres conditions encore plus préférentielles, le procédé ci-dessus décrit est mis en oeuvre de la manière suivante :
- à partir de l'acide hydroxymandélique correspondant (II avec M égal à H),
- en présence de 0,10 ± 0,02 mole de disulfite de sodium par mole d'acide hydroxymandélique correspondant mis en oeuvre au départ.
- en présence de 1,1 ± 0,1 mole d'acide formique par mole d'acide hydroxymandélique correspondant mis en oeuvre au départ.

L'acide hydroxymandélique, II avec M = H, peut être préparé extemporanément dans le milieu réactionnel à partir d'un de ses sels de métal alcalin ou d'ammonium par l'action de la quantité stoechiométrique d'un acide minéral ou organique tel que l'acide chlorhydrique, l'acide sulfurique, l'acide orthophosphorique.

Parmi les acides hydroxyphénylacétiques de formule (I), on peut citer notamment :
- l'acide orthohydroxyphénylacétique,
- l'acide parahydroxyphénylacétique,
- l'acide hydroxy-4 méthoxy-3 phénylacétique,
- l'acide chloro-4 hydroxy-2 phénylacétique.

En fin de réaction, l'acide hydroxyphénylacétique de formule (I) est isolé du milieu réactionnel par des moyens connus en soi. Parmi ces derniers moyens, un des plus simples consiste à concentrer le milieu réactionnel, acidifié à un pH inférieur ou égal à 2 avec un acide minéral, sous pression réduite, jusqu'à cristallisation de l'acide recherché que l'on isole ensuite par filtration.

On peut également extraire l'acide cherché avec un solvant convenable insoluble dans l'eau tel que l'acétate d'éthyle, l'oxyde de diéthyle.

Les produits de départ de formule (II) sont soit des produits connus, soit des produits aisément accessibles par des procédés connus tels que la condensation du cyanure de sodium sur l'hydroxybenzaldéhyde correspondant suivie de l'hydrolyse de l'hydroxymandélonitrile obtenu.

Les exemples suivants illustrent la présente invention sans toutefois la limiter.

### EXEMPLE 1

On dissout 104 g (0,5 mole) de parahydroxymandélate de sodium pur cristallisé avec une molécule d'eau dans 400 g d'eau, puis on introduit sous agitation :
- 57,6 g (o,5 mole) d'acide orthophosphorique en solution aqueuse à 85 %,
dans la solution obtenue on introduit sous agitation :
- 7,6 g (40 mmoles) de métabisulfite de sodium pur (disulfite de sodium), puis,
- 25,3 g (0,55 mole) d'acide formique à 98-100 %.

Cette solution est ensuite chauffée dans un autoclave sous agitation pendant 9 heures à 120°C sous une pression de 5 bars.

On refroidit ensuite le milieu réactionnel à la température ambiante et puis on l'amène à la pression atmosphérique. Dans la solution obtenue, on dose par chromatographie liquide à hautes performances, HPLC, 74,5 g d'acide parahydroxyphénylacétique, désigné ci-après, APHPA, soit un rendement de 98 % de la théorie calculée par rapport au parahydroxymandélate de sodium mis en oeuvre.

On acidifie le milieu réactionnel à pH = 2 avec 15 g (0,13 mole) d'acide orthophosphorique en solution aqueuse à 85 %, puis on élimine 200 g d'eau par distillation sous pression réduite. Par refroidissement du milieu réactionnel à la température ambiante, l'acide parahydroxyphénylacétique attendu cristallise spontanément. On obtient un premier jet de 55,3 g (0,3635 mole) d'acide parahydroxyphénylacétique pur cristallisé présentant un point de fusion de 150°C. Par concentration des eaux mères, on isole un deuxième jet de 13,5 g (0,09 mole) qui après recristallisation dans de l'eau conduit à de l'APHPA pur.

### EXEMPLE 2

On chauffe sous agitation dans un autoclave pendant 12 heures à 100°C sous une pression de 2 bars une solution constituée par :
- 104 g (0,5 mole) de parahydroxymandélate de sodium pur cristallisé avec une molécule d'eau,
- 400 g d'eau,
- 25,3 g (0,55 mole) d'acide formique pur,
- 7,6 g (40 mmoles) de métabisulfite de sodium pur.

En fin de réaction, on dose dans la solution réactionnelle par HPLC, 46,4 g d'APHPA soit un rendement de 61 % de la théorie calculée par rapport au parahydroxymandélate de sodium mis en oeuvre.

### EXEMPLE 3

On reproduit l'exemple 2, mais on ne chauffe que 9 heures à 100°C sous une pression de 5 bars. En fin de réaction, on dose dans la solution réactionnelle 45,6 g d'APHPA (rendement 60 %).

### EXEMPLE 4

On reproduit l'exemple 1, mais on ne chauffe que 9 heures à 100°C sous une pression de 5 bars. En fin de réaction, on dose dans la solution réactionnelle 69,2 g d'APHPA (rendement 91 %).

### EXEMPLE 5

Dans un autoclave, on chauffe pendant 8 heures à 100°C, sous agitation, sous une pression de 5 bars :
- 104 g (0,5 mole) de parahydroxymandélate de sodium cristallisé avec une molécule d'eau,
- 23 g (0,5 mole) d'acide formique pur,
- 57,6 g (0,5 mole) d'acide orthophosphorique à 85% dans l'eau,
- 10,4 g (0,06 mole) de dithionite de sodium pur,
- 400 g d'eau.

En fin de réaction, on dose dans le milieu réactionnel 0,425 mole d'acide parahydroxyphénylacétique (rendement 85 %).

## Revendications

1. Procédé de préparation d'un acide hydroxyphénylacétique de formule (I) 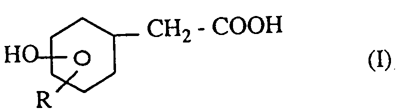 dans laquelle R représente un atome d'hydrogène, d'halogène ou un groupement alcoyle ou alcoxyle ainsi que de ses sels, caractérisé par le fait que l'on fait réagir, en milieu aqueux, à une température supérieure ou égale à 50°C, l'acide hydroxymandélique correspondant, libre ou salifié, de formule (II) 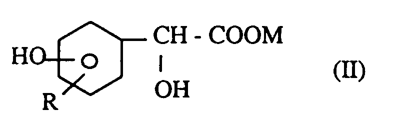 dans laquelle R a la signification donnée précédemment et M représente un atome d'hydrogène, de sodium, de potassium ou un groupement ammonium, avec de l'acide formique, en présence de quantités catalytiques d'un dérivé oxygéné du soufre présentant un degré d'oxydation de 2 à 4, pour obtenir l'acide hydroxyphénylacétique de formule (I) que l'on isole ou, si désiré, l'on salifie selon les méthodes usuelles.

2. Procédé selon la revendication 1, caractérisé par le fait que le dérivé oxygéné soufré présentant un degré d'oxydation de 2 à 4 est choisi dan le groupe constitué par le dioxyde de soufre, l'hydrogènosulfite de sodium ou de potassium, le sulfite de sodium ou de potassium, le disulfite de sodium ou de potassium, le dithionite de sodium ou de potassium, le thiosulfate de sodium ou de potassium.

3. Procédé selon l'une quelconque des revendications 1 et 2, caractérisé par le fait qu'il est effectué à une température comprise entre 75 et 150°C.

4. Procédé selon l'une quelconque des revendications 1 à 3, caractérisé par le fait qu'il est effectué sous une pression de 2 à 10 bars.

5. Procédé selon l'une quelconque des revendications 1 à 4, caractérisé par le fait que le produit de départ de formule (II) est l'acide hydroxymandélique de formule (II) dans laquelle M représente un atome d'hydrogène.

6. Procédé selon l'une quelconque des revendications 1 à 5, caractérisé par le fait qu'il est effectué en présence de 1,1 ± 0,1 mole d'acide formique par mole de produit de départ.

7. Procédé selon l'une quelconque des revendications 1 à 6, caractérisé par le fait qu'il est effectué sous une pression de 4 à 5 bars et à une température de 100 à 130°C.

8. Procédé selon l'une quelconque des revendications 1 à 7, caractérisé par le fait qu'il est effectué en présence de quantités catalytiques de disulfite de sodium.

9. Procédé selon l'une quelconque des revendications 1 à 8, caractérisé par le fait qu'il est effectué en présence de 0,10 ± 0,02 mole de disulfite de sodium par mole de produit de départ.

## Claims

1. Preparation process for a hydroxyphenylacetic acid of formula (I): 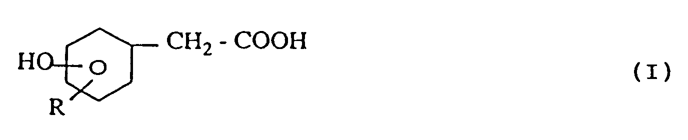 in which R represents a hydrogen atom, a halogen atom or an alkyl or alkoxyl group as well as its salts, characterized in that the corresponding free or salified hydroxymandelic acid of formula (II): 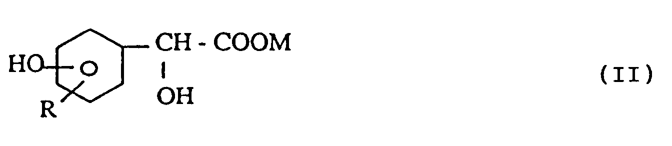 in which R has the meaning given previously and M represents a hydrogen, sodium, potassium atom or an ammonium group, is reacted in an aqueous medium, at a temperature greater than or equal to 50°C, with formic acid in the presence of catalytic quantities of an oxygenated derivative of sulphur having an oxidation number from 2 to 4, in order to obtain the hydroxyphenylacetic acid of formula (I) which is isolated or, if desired, is salified according to the usual methods.

2. Process according to claim 1, characterized in that the oxygenated derivative of sulphur having an oxidation number from 2 to 4, is chosen from the group constituted by sulphur dioxide, sodium or potassium hydrogen sulphite, sodium or potassium sulphite, sodium or potassium disulphite, sodium or potassium dithionite, sodium or potassium thiosulphate.

3. Process according to any one of claims 1 and 2, characterized in that it is carried out at a temperature comprised between 75 and 150°C.

4. Process according to any one of claims 1 to 3, characterized in that it is carried out under a pressure of 2 to 10 bars.

5. Process according to any one of claims 1 and 4, characterized in that the starting product of formula (II) is hydroxymandelic acid of formula (II) in which M represents a hydrogen atom.

6. Process according to any one of claims 1 to 5, characterized in that it is carried out in the presence of 1.1 ± 0.1 mole of formic acid per mole of starting product.

7. Process according to any one of claims 1 to 6, characterized in that it is carried out under a pressure of 4 to 5 bars and at a temperature of 100 to 130°C.

8. Process according to any one of claims 1 to 7, characterized in that it is carried out in the presence of catalytic quantities of sodium disulphite.

9. Process according to any one of claims 1 and 8, characterized in that it is carried out in the presence of 0.10 ± 0.02 mole of sodium disulphite per mole of starting product.

## Patentansprüche

1. Verfahren zur Herstellung einer Hydroxyphenylessigsäure der Formel (I) 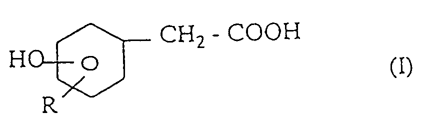 in welcher R ein Wasserstoffatom, ein Halogenatom oder eine Alkyl- oder Alkoxylgruppe bedeutet, sowie von deren Salzen, dadurch gekennzeichnet, daß man in wäßrigem Medium bei einer Temperatur über oder gleich 50°C die entsprechende freie oder in ein Salz umgewandelte Hydroxymandelsäure der Formel (II) 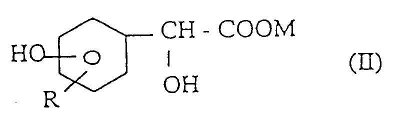 in welcher R die oben angegebene Bedeutung hat und M ein Wasserstoff-, Natrium- oder Kaliumatom oder eine Ammoniumgruppe bedeutet, mit Ameisensäure in Gegenwart katalytischer Mengen eines Sauerstoffderivats von Schwefel, das einen Oxidationsgrad von 2 bis 4 aufweist, zur Erzielung der Hydroxyphenylessigsäure der Formel (I) umsetzt, die man isoliert oder gegebenenfalls nach herkömmlichen Verfahren in ein Salz umwandelt.

2. Verfahren nach Anspruch 1, dadurch gekennzeichnet, daß das Sauerstoffderivat von Schwefel mit einem Oxidationsgrad von 2 bis 4 aus der Gruppe ausgewählt ist, die aus Schwefeldioxid, Natrium- oder Kaliumhydrogensulfit, Natrium- oder Kaliumsulfit, Natrium- oder Kaliumdisulfit, Natrium- oder Kaliumdithionit oder Natrium- oder Kaliumthiosulfat besteht.

3. Verfahren nach irgendeinem der Ansprüche 1 und 2, dadurch gekennzeichnet, daß es bei einer Temperatur zwischen 75 und 150°C durchgeführt wird.

4. Verfahren nach irgendeinem der Ansprüche 1 bis 3, dadurch gekennzeichnet, daß es unter einem Druck von 2 bis 10 bar durchgeführt wird.

5. Verfahren nach irgendeinem der Ansprüche 1 bis 4, dadurch gekennzeichnet, daß das Ausgangsprodukt der Formel (II) Hydroxymandelsäure der Formel (II) ist, in welcher M ein Wasserstoffatom bedeutet.

6. Verfahren nach irgendeinem der Ansprüche 1 bis 5, dadurch gekennzeichnet, daß es in Gegenwart von 1,1 ± 0,1 mol Ameisensäure pro mol Ausgangsprodukt durchgeführt wird.

7. Verfahren nach irgendeinem der Ansprüche 1 bis 6, dadurch gekennzeichnet, daß es unter einem Druck von 4 bis 5 bar und bei einer Temperatur von 100 bis 130°C durchgeführt wird.

8. Verfahren nach irgendeinem der Ansprüche 1 bis 7, dadurch gekennzeichnet, daß es in Gegenwart katalytischer Mengen von Natriumdisulfit durchgeführt wird.

9. Verfahren nach irgendeinem der Ansprüche 1 bis 8, dadurch gekennzeichnet, daß es in Gegenwart von 0,10 ± 0,02 mol Natriumdisulfit pro mol Ausgangsprodukt durchgeführt wird.
